# EUROPEAN PATENT APPLICATION

(11) **EP 2 815 709 A1**
(43) Date of publication of application: **24.12.2014**
(21) Application number: 14170869.3
(22) Date of filing: 03.06.2014
(51) Int. Cl.: A61B 17/68, A61B 17/86

(54) **Fracture treatment device**

(30) Priority: 17.06.2013 JP 2013126450
(71) Applicant: GC Corporation, Bunkyo-ku Tokyo 113-0033 (JP)
(72) Inventor: Yamanaka, Katsuyuki, Tokyo, 174-8585 (JP); Sakai, Yuuhiro, Tokyo, 174-8585 (JP); Yamamoto, Katsushi, Tokyo, 174-8585 (JP); Shigemitsu, Yusuke, Tokyo, 174-8585 (JP); Kaneko, Tadashi, Tokyo, 174-8585 (JP)
(74) Representative: Beck Greener

(57) **Abstract**

Provided is a fracture treatment device enabling efficient bone regeneration. The fracture treatment device (10) for connecting a bone on one side of a fracture site and a bone on the other side of the fracture site, the device including a fixation member (11) having a stick shape, a tissue regeneration structure (15) disposed in a manner to cover at least a part of the fixation member, wherein the tissue regeneration structure includes a support body made of a bioabsorbable material and cells retained in the support body, which cells regenerate bone.

## Description

### Technical Field

The present invention relates to a fracture treatment device to be used for medical treatment for a fracture site caused by bone fracture, osteotomy or the like.

### Background Art

For a fracture of femoral neck for example, in order to prevent a patient from being bedridden for a long time or to prevent false joint or necrosis, in many cases, a treatment by operation is carried out. In this treatment by operation, a plate or an external fixator to connect and fixate a bone on one side of the fracture site and a bone on the other side of the fracture site is used to join them, or a screw, a nail, an intramedullary nail, and combination of a plurality of those as a fixation tool are used to penetrate the marrow thereby joining the bones (for example, Patent Document 1 (Japanese Patent Application Laid-Open No. H08-126650) and Patent Document 2 (Japanese Patent Application Laid-Open No. H05-184597)).

Also, in some cases, the screw, the nail, the intramedullary nail, and the fixation tool are used in the same way as above for joining bones at a fracture site of other bones than femur and joining bones at a site where an osteotomy was needed for treatment.

### Summary of the Invention

### Problems to be Solved by the Invention

However, in the treatment carried out by the conventional means described above, the fixation tool such as the plate, screw, and nail used in the treatment cannot be removed in many cases. In such cases, these means are left being fixated inside the body. One reason of this is that there are problems in bone regeneration, for example, in some cases, bone is not regenerated at a fracture site caused by bone fracture or osteotomy, and bone regeneration is slow.

Accordingly, an object of the present invention is to provide a fracture treatment device with which a bone can be efficiently regenerated.

### Means for Solving the Problems

Hereinafter, the present invention will be described. In order to make the present invention easy to understand, reference numerals given in the accompanying drawings are shown here in parentheses. However, the present invention is not limited to this.

A first aspect of the present invention is a fracture treatment device (10) for bone connecting, the device comprising a fixation member having a stick shape (11) and a tissue regeneration structure (15) to be disposed in a manner to cover at least a part of an outer periphery of the fixation member, wherein the tissue regeneration structure includes a support body made of a bioabsorbable material and cells retained in the support body, the cells regenerating bone.

A second aspect of the present invention is the fracture treatment device (10) according to the first aspect, wherein a screw is formed on a portion (13, 14) of the fixation member (11), the portion not being covered by the tissue regeneration structure (15).

A third aspect of the present invention is the fracture treatment device (10) according to the first or second aspect, wherein the cells retained in the support body are chondrocytes or stem cells that differentiate into chondrocytes.

A fourth aspect of the present invention is the fracture treatment device (10) according to the first or second aspect, wherein the cells retained in the support body are stem cells that differentiate into chondrocytes and the stem cells are mesenchymal stem cells.

### Effects of the Invention

According to the present invention, it is possible to, while firstly joining a fracture site by means of the fixation member, promote bone regeneration by the tissue regeneration structure, thereby carrying out a treatment to regenerate bone tissue.

### Brief Description of the Drawings

Fig. 1A is an external view of a fracture treatment device 10;
Fig. 1B is a view of the fracture treatment device 10 showing the part of a tissue regeneration structure 15 in cross section;
Fig. 2A is a view of a fracture treatment device 10' showing the part of the tissue regeneration structure 15 in cross section;
Fig. 2B is a view of a fracture treatment device 10" showing the part of the tissue regeneration structure 15 in cross section;
Fig. 3 is a view describing one example of treatment with the fracture treatment device 10;
Fig. 4 is a view describing the example of treatment with the fracture treatment device 10;
Fig. 5 is a view describing the example of treatment with the fracture treatment device 10;
Fig. 6 is a view describing the example of treatment with the fracture treatment device 10;
Fig. 7 is a view describing the example of treatment with the fracture treatment device 10;
Fig. 8 is a view describing the example of treatment with the fracture treatment device 10;
Fig. 9 is a view describing another example of treatment with the fracture treatment device 10.

### Modes for Carrying Out the Invention

The functions and benefits of the present invention will be apparent from the following embodiments for carrying out the invention. However, the present invention is not limited to these embodiments.

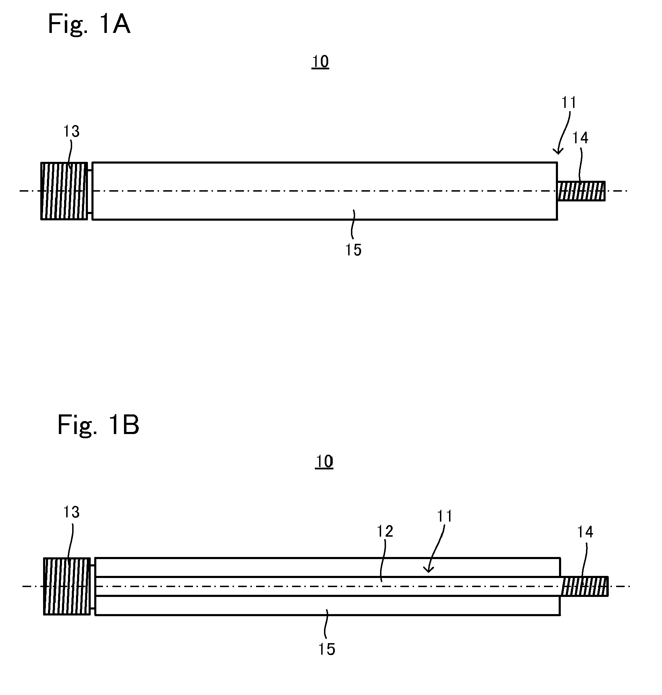

Fig. 1 includes views describing one embodiment. Fig. 1A is an external view of a fracture treatment device 10, and Fig. 1B is a cross-sectional view of the fracture treatment device 10 taken along a longitudinal direction of the fracture treatment device 10. In the cross-sectional view of Fig. 1B, only a tissue regeneration structure 15 is shown in cross section.

As can be seen from Figs. 1A and 1B, the fracture treatment device 10 includes a fixation member 11 and the tissue regeneration structure 15.

The fixation member 11 is a stick-shaped member mechanically for connecting a bone on one side of a fracture site and a bone on the other side of the fracture site to join them, and configured including a main body portion 12, a first end portion 13, and a second end portion 14.

The main body portion 12 is a member having a long slender cylindrical shape. Its length can be set with a same viewpoint as in setting length of a normal screw for treatment of fracture. In this embodiment, the first end portion 13 is disposed on one end side of the main body portion 12 in the longitudinal direction, and the second end portion 14 is disposed on the other end side.

As can be seen from Fig. 1B, the first end portion 13 is provided to one end side of the main body portion 12, concentric with a central axis of the cylindrical shape of the main body portion 12. The first end portion 13 is a cylindrical portion and its diameter is formed larger than that of the main body portion 12. Preferably, the diameter of the first end portion 13 is nearly same as an outer diameter of the tissue regeneration structure 15 described below. This enables the tissue regeneration structure 15 and a target site to have close contact with each other more appropriately, when the fracture treatment device 10 is applied to the target site, and whereby it is possible for the bones to regenerate more efficiently.

Also, it is preferable that a screw is formed on an outer peripheral portion of the first end portion 13 having a cylindrical shape. This fixates more firmly one end of the fracture treatment device 10 and the bone on the one side of the fracture site. Further, it is preferable that the first end portion 13 is provided with an engaging part (not shown) with which a tool can be engaged, such as a cross slot and a hexagonal slot to rotate the fixation member 11 on the axis of the fixation member 11. This makes it possible for the fixation member 11 to easily attach and remove to/from the target site.

As can be seen from Fig. 1B, the second end portion 14 is a part provided to the other end side of the main body portion 12, the opposite side from the first end portion 13, concentric with a central axis of the cylindrical shape of the main body portion 12.

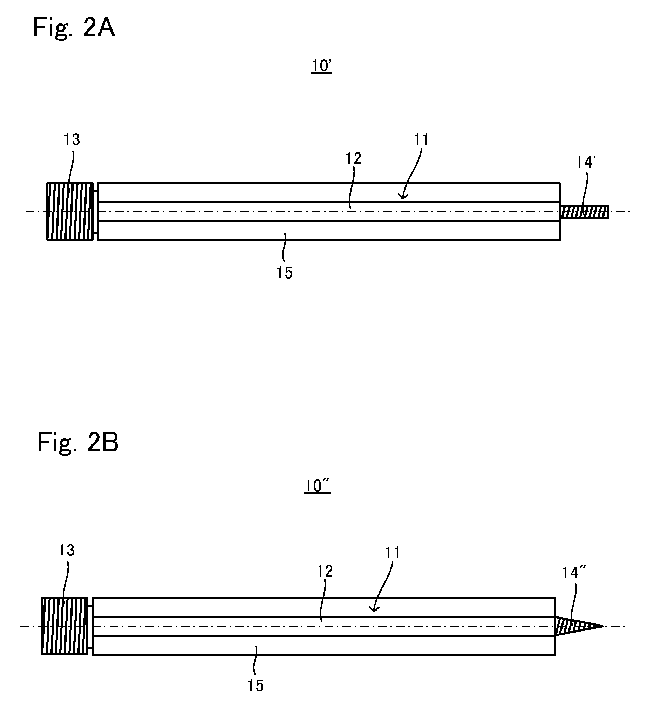

The second end portion 14 can be formed so as to have a same size in diameter as the main body portion 12 as shown in this embodiment. Alternatively, as can be seen in a second end portion 14' shown in the view of the fracture treatment device 10' of Fig. 2A, in which the tissue regeneration structure 15 is shown in cross section, the second end portion 14' can be formed thinner than the main body portion 12. Further, as in a second end portion 14" shown in the view of the fracture treatment device 10" of Fig. 2B, in which the tissue regeneration structure 15 is shown in cross section, the second end portion 14" can be formed so as to be tapered from a side of the main body portion 12 to a tip.

It is preferable that a screw is formed on an outer peripheral portion of the second end portion 14. This fixates more firmly the other end of the fracture treatment device 10 and the bone on the other side of the fracture site.

The fixation member 11 preferably has a biocompatibility together with a predetermined strength. From this viewpoint, the fixation member 11 is preferably made of titanium or titanium alloy.

The tissue regeneration structure 15 is a structure contributing to bone regeneration, and a member in which cells are retained in its support body.

The support body is a member configured so as to be enable to retain required cells, and in this embodiment, it is formed in a cylindrical shape as a whole. As described below, the main body portion 12 of the fixation member 11 is disposed inside of the support body having a cylindrical shape. Therefore, the inner diameter of the support body is preferably nearly same as the diameter of the main body portion 12.

Material of the support body to retain the cells is not particularly limited as long as the material is safe to biological body and bioabsorbable, and the support body can have a structure as follows for example.

The support body can be made of a bioabsorbable polymer material and have a communicating pore structure, wherein each pore has a pore diameter of 5 µm or more to 3500 µm or less (5 µm ≤ pore diameter ≤ 3500 µm), an average pore diameter is 50 µm or more to 2000 µm or less (50 µm ≤ average pore diameter ≤ 2000 µm), and a porosity is 60% or more to 95% or less (60% ≤ porosity ≤ 95%). Here, the average pore diameter means a number average pore diameter, and micro pores each having a diameter of less than 10 µm are not considered in calculation. Also, the "porosity" is a value calculated from a weight of the support body to a weight of the used law material lump having a same volume as the support body. If the porosity is less than 60%, culture efficiency of chondrocytes or stem cells that differentiate into chondrocytes possibly degrades, and if the porosity is more than 95%, the strength of the support body itself possibly degrades. Therefore, the porosity is more preferably 80% or more to 90% or less (80% ≤ porosity ≤ 90%).

The pore diameter is preferably 180 µm or more to 3500 µm or less (180 µm ≤ pore diameter ≤ 3500 µm). If the pore diameter is less than 180 µm, it is difficult to introduce the cells into the support body, whereby there is a possibility that the cells cannot be retained (seeded) sufficiently to inside the support body. On the other hand, if the pore diameter is more than 3500 µm, the strength of the support body itself tends to degrade.

Also, the average pore diameter is preferably 540 µm or more to 1200 µm or less (540 µm ≤ average pore diameter ≤ 1200 µm).

Also, the support body is preferably configured to have a compressive strength of 0.05 MPa or more to 1 MPa or less (0.05 MPa ≤ compressive strength ≤ 1 MPa). If the compressive strength is less than 0.05 MPa, there is a possibility that the support body shrinks because of extension stress of the cells. On the other hand, it is technically difficult to make a support body having a compressive strength of more than 1 MPa. The "compressive strength" means a compressive breaking strength when a specimen formed in a cylindrical shape having a diameter of 10 mm and a height of 2 mm is compressed at a crosshead speed of 1 mm/min.

The material to constitute the support body may be any without particular limitations as long as it is a bioabsorbable polymer material as described and can maintain its configuration inside the body for a certain period. Examples thereof include at least one kind selected from the group consisting of polyglycolic acid, polylactic acid, a copolymer of lactic acid and glycolic acid, poly-s-caprolactone, a copolymer of lactic acid and ε-caprolactone, polyamino acid, polyortho ester and copolymer thereof. Among these, polyglycolic acid, polylactic acid and a copolymer of lactic acid and glycolic acid are most preferred as approved by U.S. Food and Drug Administration (FDA) as a polymer harmless to human body and in view of their actual performance. The weight average molecular weight (M) of the bioabsorbable polymer material is preferably 5000 or more to 2000000 or less (5000 ≤ M ≤ 2000000), more preferably 10000 or more to 500000 or less (10000 ≤ M ≤ 500000).

By applying such a support body, a culture solution containing the cells can be appropriately permeated inside the support body more certainly, whereby it is possible to introduce and retain the cells to be retained in the support body stably and without any waste, to produce the tissue regeneration structure 15.

Production method of the support body is not particularly limited. However, the production method may be for example as follows: mixing, in a substantially uniform manner, a particulate substance having a particle diameter of 100 to 2000 µm into an organic solvent in which the bioabsorbable polymer material is dissolved and freezing the mixture, the particle substance not dissolving in this organic solvent but dissolving in a liquid that does not dissolve the bioabsorbable polymer material; thereafter drying the mixture to remove the organic solvent, thereby producing a porous bioabsorbable polymer material having a pore structure with a pore diameter of 5 to 50 µm and contains the particulate substance; pulverizing the porous bioabsorbable polymer with a mill or the like; then removing the particulate substance by dissolving it in the liquid that does not dissolve the bioabsorbable polymer; thereafter sifting it to make a granular bioabsorbable porous substance having an average particle diameter of 100 µm or more to 3000 µm or less (100 µm ≤ average particle diameter ≤ 3000 µm) ; and then putting the granular bioabsorbable porous substance into a container having a predetermined shape to pressurize and heat it.

The cells to be retained in the support body are cells that can regenerate bones, and the support body retains the culture solution containing the cells.

The cells to be used in the present invention are preferably chondrocytes that are to be bone cells in the end or that promote formation of bone tissue, or stem cells that differentiate into chondrocytes (hereinafter, sometimes referred to as "chondrocyte differentiating stem cell"). Examples of the chondrocyte differentiating stem cells include cells that can differentiate into chondrocytes, such as marrow-derived mesenchymal stem cells, fat-derived mesenchymal stem cells, mesenchymal cells and synovial cells, or cells capable of promoting bone repairing. Examples of such cells include cells harvested from: a bone marrow of a pelvis (an iliac bone) or of long bones of arms and legs (a thighbone, a tibia); and/or a bone marrow of periosteum, synovium, fat, alveolar bone and the like; a periosteum of a palate, an alveolar bone and the like; or other sources.

As a method to harvest these cells, methods normally carried out in the medical field can be employed without particular limitations. In this regard, it is preferable that the cells are harvested from places such as the bone marrow of ilium and the like, and the periosteum of palate, alveolar bone and the like, since it requires only an easy operation to harvest the cells, and invasion such as cutting and peeling of the skin and the muscle can be reduced.

The chondrocytes and the chondrocyte differentiating stem cells can be harvested not only from a person to be applied, but also from another person dead or alive, or animals such as bovine, porcine, equine, and avian. However, when taking the cells from another person or an animal, in consideration of the possibility that the immunological rejection may occur, it is necessary to carry out an antigen removal treatment such as decellularization through rapid freezing by liquid nitrogen. Such a treatment can reduce damage to the person to be applied.

The harvested chondrocytes or chondrocyte differentiating stem cells are amplified by being cultivated for one to two weeks in a culture container for tissue culture by a known method. A known culture medium can be used for the culture, and especially α-MEM for cell culture containing autoserum and fetal bovine serum can be preferably used. In a case where the mesenchymal cells are applied, by causing an action of a specific growth factor (for example, bFGF) to occur, the mesenchymal cells increase with their high multipotency being kept, and whereby it is possible to promote differentiation of cartilage.

The tissue regeneration structure 15 as described above can be produced for example as follows. That is, after filling the entirety of inside of the communicating pore structure of the support body with an inducing aqueous solution, supplying the culture solution in which the cells produced as described above are suspended to a part of the support body, and at the same time, drawing the inducing aqueous solution from another part of the support body. This causes a negative pressure between the inducing aqueous solution being drawn from another part of the support body and the culture solution supplied to a part of the support body, in which the cells are suspended. The supplied culture solution follows the flow of the inducing aqueous solution being drawn and spreads all over the inside of the communicating pore structure of the support body in a manner to switch with the inducing aqueous solution. In this way, the tissue regeneration structure 15 can be produced. More specifically, the tissue regeneration structure 15 can be produced as follows.

First, the entirety of inside of the communicating pore structure of the prepared support body is filled with the inducing aqueous solution. This inducing aqueous solution is an aqueous solution used to be switched with the culture solution in which the cells are suspended. As the inducing aqueous solution, water, normal saline, buffer solution, biological fluid, culture solution and the like can be used. Since the inducing aqueous solution does not contain the cells, it can be forcibly introduced to the support body by being pressured and the like. For example, by immersing the support body in the inducing aqueous solution and reducing the pressure of the support body, the support body can be saturated with the inducing aqueous solution to inside thereof.

As shown above, after filling the entirety of inside of the communicating pore structure with the inducing aqueous solution, the culture solution in which the cells are suspended as described above is supplied to some part of the support body, and at the same time, the inducing aqueous solution is drawn from another part of the support body. This supply of the culture solution in which the cells are suspended to some part of the support body and the drawing of the inducing aqueous solution from another part can be carried out substantially at the same time. Alternatively, the drawing can be started on ahead from another part of the support body, and before a large amount of the inducing aqueous solution is drawn, immediately the culture solution in which the cells are suspended can be supplied to some part of the support body. Also, if the remained part from the part where the culture solution is supplied and the part where the inducing aqueous solution is drawn is framed, it is possible to prevent the culture solution in which the cells are suspended from flowing out from the support body.

Means for drawing the inducing aqueous solution from the support body is not particularly limited, however, preferably the means is a water absorber having contact with the support body, since it is possible to draw easily the inducing aqueous solution without using a complicated suction equipment, and the solution is not excessively drawn, therefore the cells are not heavily damaged. As the water absorber, any material can be used without particular limitations, as long as the material is capable of appropriately absorbing the inducing aqueous solution from the support body. For example, cellulose-based or paper-based filter papers, water absorbent polymer materials, porous blocks made of inorganic or organic material and the like can be used. Among these, it is preferred to combine one kind or two kinds or more selected from the group consisting of: various papers such as filter papers, paper towels, blotting papers, processed papers; various fibers such as cotton, silica wool, silk, wool, glass wool, rayon, hemp, cellulose acetate, cellulose nitrate; various porous absorbing materials such as silica gel, diatomite, cellulose powder; and water absorbent polymer materials, since these are easily obtained and used.

In order to adjust cell concentration in the support body, the supply and drawing as described above can be repeated more than once to the solution in the support body, in which the cells are suspended after drawing, after predetermined period.

The support body produced as above, in which the cells are retained, can be made as the tissue regeneration structure 15. Further, in the support body in which the cells are retained, at least a part of a surface to be in contact with the bone of the target site can be covered with an engraftment layer being a layer having a high cell concentration without having the support body. Since the engraftment layer does not have the support body and has a high cell concentration, engraftment to the bone can be further promoted. The engraftment layer can be, for example, produced by layering a base material in a gel form having a high cell concentration to a part of the support body produced as above and in which the cells are retained. As the material of the base material: extracellular matrix protein (matrigel and the like) such as fibrin (blood plasma fibrin in which molecular is enlarged by resolidification reaction (including PRP (Platelet Rich Plasma), PPP (Platelet Poor Plasma) and fibrin glue)), gelatin, and collagen; artificial protein; and peptide can be used as an aqueous solution (normally, by adding to the culture solution). Each of these can be formed in a gel. By being formed in a gel, it can be formed not having flowability, therefore the engraftment layer can be retained stably on the tissue regeneration structure even without the support body.

The fracture treatment device 10 is configured by including the fixation member 11 and the tissue regeneration structure 15 described above. That is, as can be seen from Fig. 1B, in the fixation member 11, the main body portion 12 is inserted into the tissue regeneration structure 15 having a cylindrical shape, thereby being disposed such that the tissue regeneration structure 15 covers at least a part of an outer periphery of the main body portion 12. And as can be seen from Fig. 1A, from both ends of the tissue regeneration structure 15, the first end portion 13 and the second end portion 14 are projected.

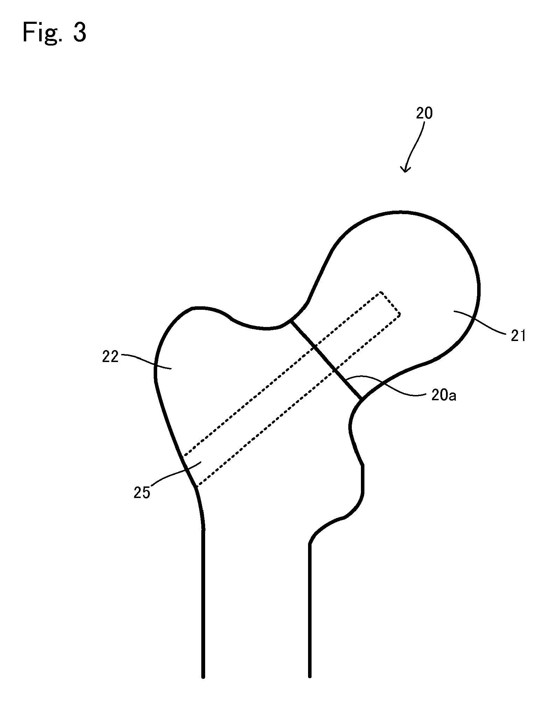

The fracture treatment device 10 is for example applied to the target site as follows. Figs. 3 to 8 are views for explanation.

Here, as shown in Fig. 3, a case where a fracture site 20a is generated between a femoral head 21 and a great trochanter 22 of a femoral neck is considered as an example.

In this case, first, a bore 25 is formed which penetrates through the fracture part 20a from a side of the great trochanter 22 to reach the middle of the femoral head 21. This bore 25 is preferably formed to have a diameter same as or slightly bigger than the outer diameter of the tissue regeneration structure 15 of the fracture treatment device 10. This enables the tissue regeneration structure 15 and an inner surface of the bore 25 to have close contact with each other, thereby regenerating the bone efficiently. Also, in a case where a screw is provided to the first end portion 13, it is preferred that the diameter of the bore 25 is taken such that the screw is screwed to the bore 25 in the vicinity of the opening portion of the bore 25. The depth of the bore 25 is preferably deep enough to be inserted with the entirety of the fracture treatment device 10.

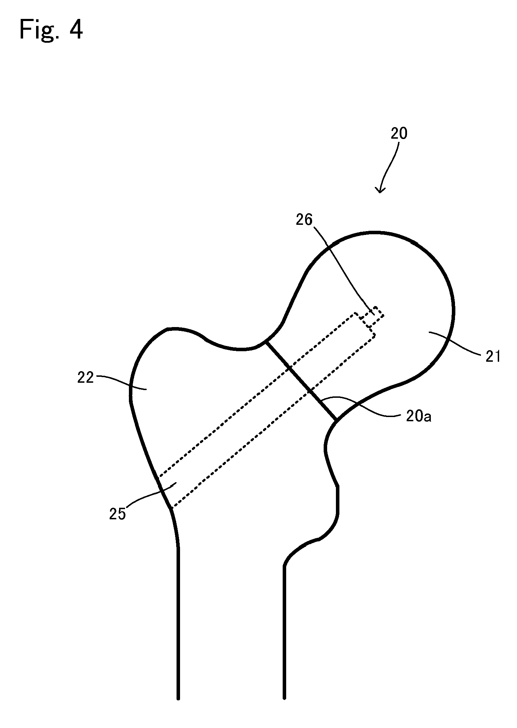

Next, as shown in Fig. 4, a bore 26 having a small diameter is formed in a manner to be extended concentrically from the deepest part of the bore 25. This bore 26 is a bore with which the second end portion 14 of the fracture treatment device 10 is to be engaged. Therefore, the bore 26 is formed such that its diameter is slightly smaller than that of the second end portion 14 so that the second end portion 14 can engage with the bore 26.

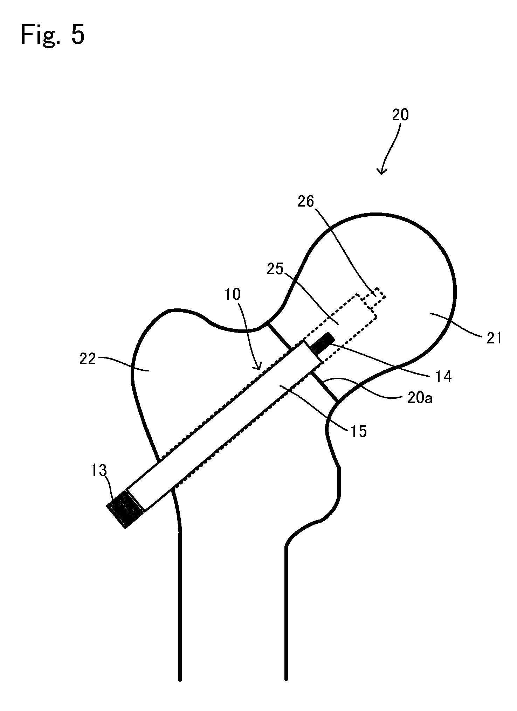

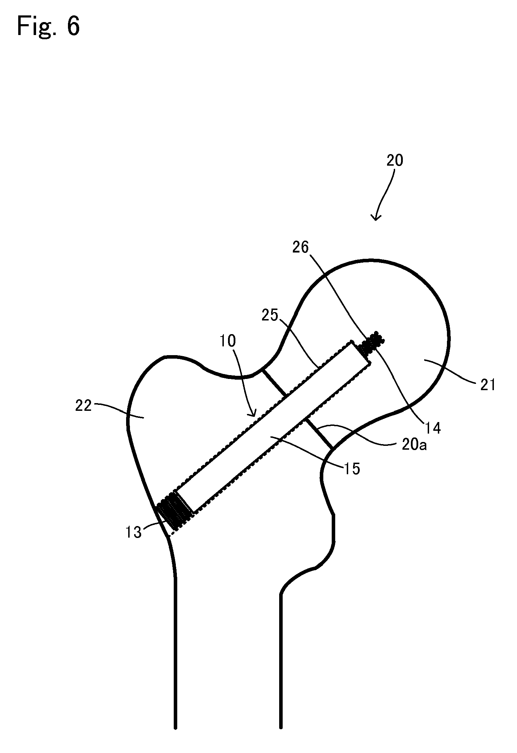

Thereafter, as shown in Fig. 5, the fracture treatment device 10 is inserted to the bore 25. At this time, the fracture treatment device 10 is inserted such that the second end portion 14 faces the bottom side of the bore 25 and the first end portion 13 faces the opening side of the bore 25. Thereafter, as shown in Fig. 6, the fracture treatment device 10 is further inserted, so that the second end portion 14 is engaged with the bore 26. On the other hand, the first end portion 13 engages with the bore 25 in the vicinity of the opening portion of the bore 25. These engagements can be formed for example by an engagement part not shown but provided to the first end portion 13 and by means of a tool to engage with the engagement part.

With the posture shown in Fig. 6, the first end portion 13 is fixated to the great trochanter 22 and the second end portion 14 is fixated to the femoral head 21, whereby both are connected by the main body portion 12.

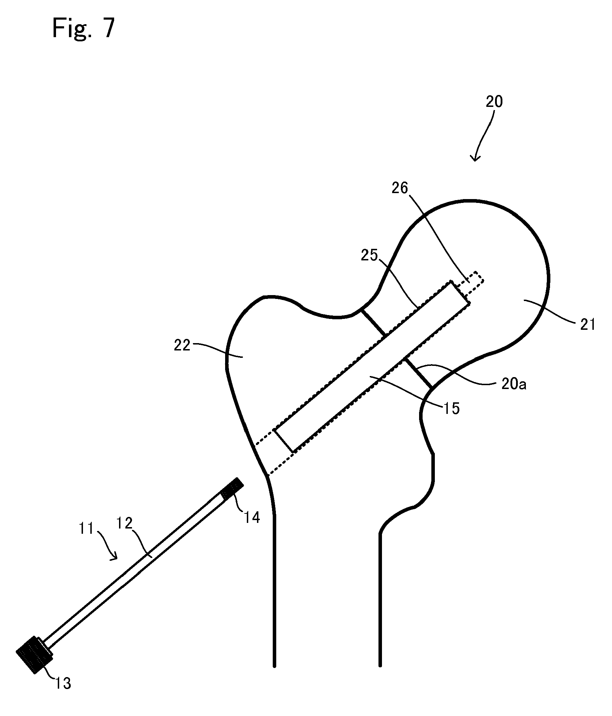

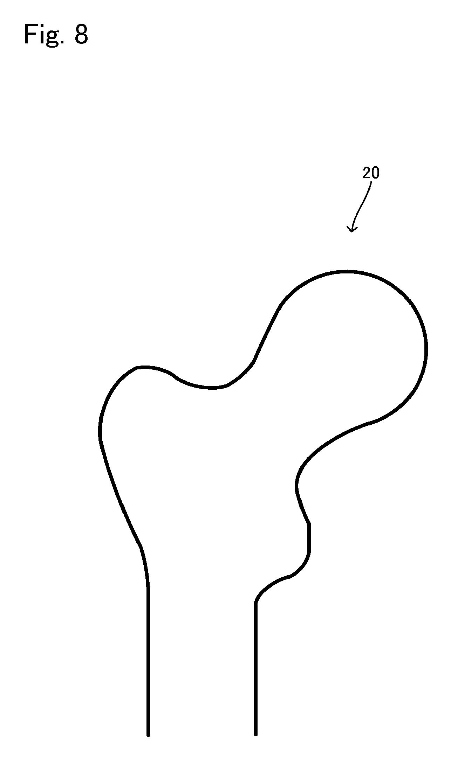

Endochondral ossification of the cells contained in the tissue regeneration structure 15 of the fracture treatment device 10 installed as described leads bone regeneration. Here, after a predetermined period passes since the fracture treatment device 10 is installed as shown in Fig. 6, only the fixation member 11 is removed from the body as shown in fig. 7. Thereafter, by going through a further period, as shown in Fig. 8, a bore formed by removing the fixation member 11 is filled because of bone regeneration, and the fracture site can be bridged by the connected bones. The support body disappears as time passes since it is formed of a bioabsorbable polymer material.

Here, an example in which one fracture treatment device 10 is used is shown, however, it is not necessary to be one, and two or more of the fracture treatment device 10 can be used.

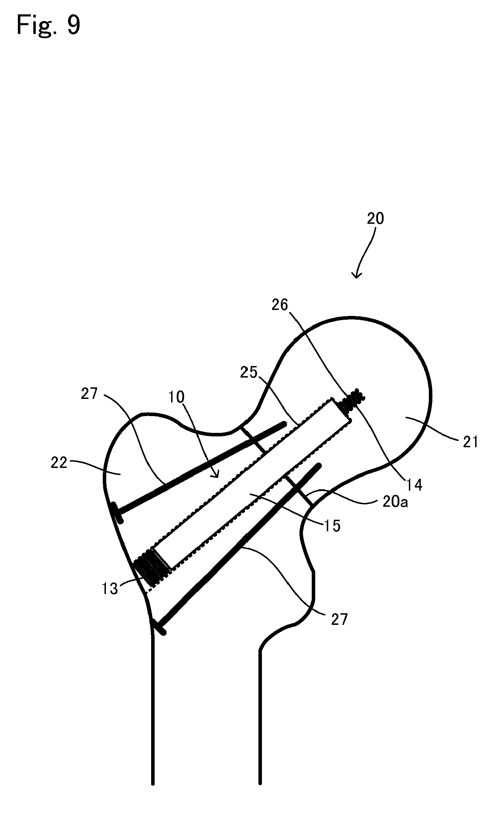

Also, as shown in Fig. 9, in addition to the fracture treatment device 10, a nail 27 can be used together as reinforcement. Fig. 9 corresponds to Fig. 6.

As described above, according to the fracture treatment device 10 of the present invention, the bones at a fracture site caused by bone fracture, osteotomy and the like are connected, and at the same time, bone regeneration is promoted, whereby it is possible to carry out a treatment of the fracture site with bone regeneration. Therefore, bones can be regenerated even in a case where bone regeneration had not been expected in the past, and devices artificially embedded in bones can be removed. Also, even when applying to a case where bone regeneration has been expected from the past, it is possible to promote bone regeneration and enables further reliable bone regeneration.

### Description of the Reference Numerals

- 10: fracture treatment device
- 11: fixation member
- 12: main body portion
- 13: first end portion
- 14: second end portion
- 15: tissue regeneration structure

## Claims

1. A fracture treatment device for bone connecting, the fracture treatment device comprising:
a fixation member having a stick shape; and
a tissue regeneration structure disposed in a manner to cover at least a part of an outer periphery of the fixation member, wherein the tissue regeneration structure includes a support body made of a bioabsorbable material and cells retained in the support body, the cells regenerating bone.

2. The fracture treatment device according to claim 1,
wherein a screw is formed on a portion of the fixation member, the portion being not covered by the tissue regeneration structure.

3. The fracture treatment device according to claim 1 or 2, wherein the cells retained in the support body are chondrocytes or stem cells that differentiate into chondrocytes.

4. The fracture treatment device according to claim 1 or 2, wherein the cells retained in the support body are stem cells that differentiate into chondrocytes and the stem cells are mesenchymal stem cells.
